# DEMANDE DE BREVET EUROPEEN

(11) **EP 4 591 889 A1**
(43) Date de publication de la demande: **30.07.2025**
(21) Numéro de dépôt: 24305145.5
(22) Date de dépôt: 26.01.2024
(51) Int. Cl.: A61K 51/04, A61K 101/02

(54) **METHODE DE DETECTION DE FIBROSE PULMONAIRE**

(71) Demandeur: Université de Bourgogne, 21078 Dijon Cedex (FR); Centre Georges François Leclerc, 21079 Dijon Cedex (FR); Centre Hospitalier Universitaire De Dijon, 21079 Dijon Cedex (FR); Institut National de la Santé et de la Recherche Médicale, 75013 Paris (FR); Institut Agro Dijon, 21079 Dijon Cedex (FR)
(72) Inventeur: BURGY, Olivier, 21540 Sombernon (FR); BELLAYE, Pierre-Simon, 21800 Quetigny (FR); BONNIAUD, Philippe, 21000 Dijon (FR); GOIRAND, Françoise, 21000 Dijon (FR)
(74) Mandataire: Gevers & Orès

(57) **Abrégé**

La présente invention concerne une méthode non invasive de détection de lésions de fibrose pulmonaire utilisant de la choline marquée. La présente invention concerne également des méthodes de diagnostic du caractère progressif, de détermination du degré de sévérité et de suivi de l'évolution d'une fibrose pulmonaire progressive basées sur cette méthode de détection. Dans un mode de réalisation préféré, la choline marquée est la [18F]-Fcholine également connue sous le nom de N-[(18F)Fluorométhyl]-2-hydroxy-N,N-diméthyléthanaminium.

## Description

### DOMAINE TECHNIQUE DE L'INVENTION

La présente invention porte sur une méthode non invasive de détection des lésions de fibrose pulmonaire. La présente invention porte également sur des méthodes de diagnostic, de détermination du degré de sévérité et de suivi de l'évolution d'une fibrose pulmonaire et de sa progression.

### ARRIERE-PLAN TECHNIQUE

Les fibroses pulmonaires (PPF) font partie du grand groupe des pneumopathies interstitielles diffuses avec de multiples étiologies. Les fibroses pulmonaires peuvent s'aggraver et on parle alors de fibrose pulmonaire progressive (PPF). Collectivement, ces PPF ont de nombreuses étiologies différentes et affectent un nombre considérable de patients (Wijsenbeek et al. Spectrum of Fibrotic Lung Diseases, N Engl J Med. 2020 Sep 3;383(10):958-968).

La fibrose pulmonaire idiopathique (FPI) est l'archétype des PPF et est associée à un déclin progressif de la fonction pulmonaire avec une médiane de survie inférieure à 5 ans après le diagnostic (Raghu G et al., Diagnosis of Idiopathic Pulmonary Fibrosis. An Official ATS/ERS/JRS/ALAT

Clinical Practice Guideline, Am J Respir Crit Care Med. 2018 Sep 1;198(5):e44-e68). La FPI affecterait environ trois millions de patients actuellement dans le monde, avec une prévalence entre 3 et 45 cas pour 100 000 dans la population générale et une incidence estimée entre 0,9 et 13 nouveaux cas pour 100 000 habitants (Martinez FJ et al., Idiopathic pulmonary fibrosis. Nat Rev Dis Primers.

2017 Oct 20;3:17074; Maher et al., Global incidence and prevalence of idiopathic pulmonary fibrosis. Respir Res. 2021; 22: 197.). Les autres fibroses progressives sont souvent associées à des maladies du tissu conjonctif comme la polyarthrite rhumatoïde ou la sclérodermie (Wijsenbeek et al., Progressive fibrosing interstitial lung diseases: current practice in diagnosis and management, Curr Med Res Opin. 2019 Nov;35(11):2015-2024).

Au niveau mécanistique la fibrose pulmonaire est la conséquence d'une cicatrisation anormale avec accumulation de composant de la matrice extracellulaire, notamment de fibres de collagène, rendant le tissu pulmonaire rigide et non fonctionnel empêchant notamment les échanges gazeux.

Les mécanismes exacts de l'initiation et de la progression de cette fibrose et des maladies associées sont encore mal compris (Burgy O. et al., Pathogenesis of fibrosis in interstitial lung disease. Curr Opin Pulm Med. 2020 Sep;26(5):429-435).

Le scanner thoracique (e.g. « high resolution-computed tomography », (HR-CT)), technique d'imagerie peu invasive, est actuellement un des examens clef pour porter le diagnostic de pneumopathie interstitielle diffuse et de la caractérisée notamment en recherchant des critères d'imagerie en faveur de fibrose (rayon de miel, bronchiectasies de traction notamment).

Néanmoins actuellement et malgré des avancées significatives des techniques du scanner il reste nécessaire d'associer à celui-ci une évaluation des symptômes et de la fonction pulmonaire pour déterminer la progression clinique de la maladie.

Cette évaluation de la fonction pulmonaire est essentiellement réalisée par mesure de la capacité vitale forcée (CVF) afin d'estimer la sévérité de la maladie. La CVF représente également une des principales données évaluées lors des essais cliniques (Behr J., Disease Progression in Idiopathic Pulmonary Fibrosis. FVC Is Not Enough, Am J Respir Crit Care Med. 2017 Nov 1;196(9):1094-1095).

Cependant, la justesse de la mesure de la CVF est limitée et dépend de plusieurs facteurs variables d'un patient à l'autre (effort du patient, sévérité de la maladie, voies aériennes obstruées lors des mesures).

En parallèle, plusieurs biomarqueurs sériques potentiels de pneumopathie interstitielle diffuse et de fibrose pulmonaire progressive ont été identifiés (Tzouvelekis A. et al., Validation of the prognostic value of MMP-7 in idiopathic pulmonary fibrosis, Respirology 2017 Apr;22(3):486-493,

Ohshimo S. et al., Baseline KL-6 predicts increased risk for acute exacerbation of idiopathic pulmonary fibrosis, Respir Med. 2014 Jul;108(7):1031-9, (Moll et al., Biomark Med.. 2020

Jul;14(11):997-1007), CXCL13 (Bellamri N. et al., TNF-α and IL-10 Control CXCL13 Expression in Human Macrophages, J Immunol. 2020 May 1;204(9):2492-2502, Qiu L. et al., A novel prognostic signature for idiopathic pulmonary fibrosis based on five-immune-related genes, Ann. Transl. Med.

2021 Oct;9(20):1570). Certains de ces biomarqueurs sont en cours de confirmation (e.g. essai clinique NCT04268485 sur le KL-6) mais aucun n'est actuellement utilisable en pratique courante en dehors de la recherche.

A ce jour, ni la mesure de la capacité vitale forcée, ni aucun biomarqueur, ni l'imagerie ne permettent un suivi précoce et quantitatif de l'évolution de la maladie ou de l'évaluation de l'efficacité des traitements.

Par conséquent, il demeure un immense besoin de développer des méthodes de diagnostic, de pronostic et de suivi des fibroses pulmonaires telles que la fibrose pulmonaire idiopathique afin d'évaluer précisément la progression.

### RESUME DE L'INVENTION

Les inventeurs ont découvert qu'une imagerie non invasive avec de la choline marquée permettait la détection du caractère progressif d'une fibrose pulmonaire, l'évaluation de sa sévérité et la prédiction de sa progression. En effet, les inventeurs ont montré que le signal émis par l'accumulation de la choline marquée, signe de l'activité de l'enzyme choline kinase, était directement et positivement corrélé à la sévérité de la fibrose pulmonaire.

La présente invention concerne donc une méthode non invasive de détection *in vivo* de lésion(s) de fibrose pulmonaire utilisant de la choline marquée comprenant une étape de quantification du signal émis par la choline marquée chez un patient dans une région déterminée, la région déterminée étant tout ou une partie des poumons du patient.

La présente invention concerne également l'utilisation de choline marquée en tant qu'agent d'imagerie afin de détecter *in vivo* des lésions de fibrose pulmonaire comprenant une étape de quantification du signal émis par la choline marquée chez un patient dans une région déterminée, la région déterminée étant tout ou une partie des poumons du patient, et une étape de génération d'une image à partir de la quantification du signal émis par la choline marquée.

L'accumulation de choline marquée au niveau pulmonaire, selon l'invention permet le diagnostic, en particulier le diagnostic du caractère progressif, la détermination du degré de sévérité et/ou le suivi de l'évolution d'une fibrose pulmonaire progressive.

Aussi, selon d'autres aspects de l'invention, un objet de la présente invention est de la choline marquée pour une utilisation dans une méthode de diagnostic *in vivo* d'une fibrose pulmonaire progressive , la méthode comprenant :
- une étape de quantification du signal émis par la choline marquée chez un patient dans une région déterminée, la région déterminée étant tout ou une partie des poumons du patient, et
- une étape de comparaison de la quantité du signal émis par la choline marquée à une valeur de référence, dite valeur de référence diagnostique,
- une quantité de signal émis par la choline marquée supérieure à la valeur de référence diagnostique étant indicatrice que le patient est atteint d'une fibrose pulmonaire progressive.

Un autre objet de l'invention est de la choline marquée pour une utilisation dans une méthode de détermination *in vivo* du degré de sévérité d'une fibrose pulmonaire progressive, la méthode comprenant :
- une étape de quantification du signal émis par la choline marquée chez un patient dans une région déterminée, la région déterminée étant tout ou une partie des poumons du patient, et
- une étape de comparaison de la quantité du signal émis par la choline marquée à au moins une valeur de référence, dite valeur de référence de grade, chaque valeur de référence de grade correspondant à un grade de sévérité donné de la fibrose pulmonaire progressive,
- une quantité de signal émis par la choline marquée supérieure ou égale à une valeur de référence de grade étant indicatrice que la sévérité de la fibrose pulmonaire progressive est au moins du grade de sévérité donné,
- une quantité de signal émis par la choline marquée inférieure à la valeur de référence de grade étant indicatrice que le degré de sévérité de la fibrose pulmonaire progressive est moindre que le grade de sévérité donné.

L'invention porte également sur de la choline marquée pour une utilisation dans une méthode de suivi *in vivo* de l'évolution d'une fibrose pulmonaire progressive, la méthode comprenant :
(a) une étape de quantification du signal émis par la choline marquée chez un patient dans une région déterminée, la région déterminée étant tout ou une partie des poumons du patient, à un temps donné T-1,
(b) une étape de quantification du signal émis par la choline marquée chez le patient dans la région déterminée à un temps donné T0, T0 étant supérieur à T-1, et
(c) la comparaison de la quantité du signal émis par la choline marquée à T0 à la quantité du signal émis par de la choline marquée à T-1,
   - une quantité du signal émis par la choline marquée à T0 supérieure à la quantité du signal émis par la choline marquée à T-1 est alors indicatrice d'une aggravation de la fibrose pulmonaire progressive chez ce patient,
   - une quantité du signal émis par la choline marquée à T0 inférieure ou égale à la quantité du signal émis par la choline marquée à T-1 est alors indicatrice d'une amélioration de fibrose pulmonaire progressive chez ce patient,
   - une quantité du signal émis par la choline marquée à T0 égale à la quantité du signal émis par la choline marquée à T-1 est alors indicatrice d'une stagnation de la fibrose pulmonaire progressive chez ce patient.

Selon un autre aspect, l'invention concerne de la choline marquée pour une utilisation dans une méthode d'évaluation *in vivo* de l'efficacité d'un traitement thérapeutique contre une fibrose pulmonaire progressive, la méthode comprenant :
(a) une étape de quantification du signal émis par la choline marquée chez un patient dans une région déterminée, la région déterminée étant tout ou une partie des poumons du patient, avant traitement thérapeutique,
(b) une étape de quantification du signal émis par la choline marquée chez le patient dans la région déterminée, après un temps donné de traitement thérapeutique et
(c) la comparaison de la quantité du signal émis par la choline marquée après un temps donné de traitement thérapeutique à la quantité du signal émis par la choline marquée avant traitement thérapeutique,
   - une diminution ou une stagnation de la quantité du signal émis par la choline marquée après un temps donné de traitement thérapeutique est indicatrice de l'efficacité du traitement thérapeutique.

### DESCRIPTION DETAILLEE DE L'INVENTION

### Méthode de détection de lésions de fibrose pulmonaire

Selon un premier aspect, l'invention concerne une méthode non invasive de détection, préférablement par imagerie, de lésion(s) de fibrose pulmonaire utilisant de la choline marquée.

L'invention concerne également de la choline marquée pour une utilisation dans une méthode non invasive de détection, préférablement par imagerie, de lésion(s) de fibrose pulmonaire.

Une ou plusieurs lésions peuvent être détectées.

L'invention concerne aussi l'utilisation de choline marquée en tant qu'agent d'imagerie afin de détecter *in vivo* une ou plusieurs lésion de fibrose pulmonaire.

L'invention concerne en outre l'utilisation de choline marquée dans la préparation d'un réactif d'imagerie pour la détection de lésion(s) de fibrose pulmonaire.

Dans tous les aspects de la présente invention, la choline marquée peut comprendre un marqueur choisi dans le groupe consistant en un marqueur fluorescent, un marqueur radioactif et un marqueur paramagnétique.

Le marqueur fluorescent peut être choisi dans le groupe consistant en un colorant polyméthine, comme les colorants dicarbocyanine, tricarbocyanine, indotricarbocyanine, mérocyanine, styryle, squarilium, cyanine holopolaire, hémicyanine, oxonol et hémioxanol ; un colorant rhodamine, un colorant phénoxazine; ou un colorant phénothiazine.

Le marqueur radioactif peut être choisi dans le groupe consistant en ²²⁵Ac, ²¹¹At, ²¹²Bi, ²¹³Bi, ⁷⁶Br, ¹¹C, ^{34m}Cl , ⁶⁷Cu, ⁶⁴Cu, ¹⁸F, ⁶⁸Ga, ¹⁶⁶Ho, ¹²³I, ¹²⁴I, ¹²⁵I, ¹³¹I, ¹³N, ²²³Ra , ¹⁸⁶Re, ¹⁸⁸Re, ⁴⁷Sc, ¹⁵³Sm, ^{94m}Tc, ^{99m}Tc, et ⁹⁰Y, ¹¹¹In, ¹⁷⁷Lu et ⁸⁹Zr.

Le marqueur paramagnétique peut être choisi dans le groupe consistant en Gd³⁺, Fe³⁺, Mn²⁺ , Yt³⁺, Dy³⁺ et Cr³⁺.

De préférence, la choline marquée comprend un marqueur radioactif, plus préférablement un marqueur radioactif choisi dans le groupe consistant en ²²⁵Ac, ²¹¹At, ²¹²Bi, ²¹³Bi, ⁷⁶Br, ¹¹C, ^{34m}Cl , ⁶⁷Cu, ⁶⁴Cu, ¹⁸F, ⁶⁸Ga, ¹⁶⁶Ho, ¹²³I, ¹²⁴I, ¹²⁵I, ¹³¹I, ¹³N, ²²³Ra , ¹⁸⁶Re, ¹⁸⁸Re, ⁴⁷Sc, ¹⁵³Sm, ^{94m}Tc, ^{99m}Tc, et ⁹⁰Y, ¹¹¹In, ¹⁷⁷Lu, ⁸⁹Zr, encore plus préférablement un marqueur radioactif choisi dans le groupe consistant en ⁷⁶Br, ¹¹C, ¹⁸F, ¹²³I, ¹²⁴I, et ¹³N, de manière la plus préférée ¹⁸F.

Selon le mode de réalisation préféré de l'invention, la choline marquée est une [¹⁸F]-Fcholine.

La [¹⁸F]-Fcholine également connue sous le nom de N-[(¹⁸F)Fluorométhyl]-2-hydroxy-N,N-diméthyléthanaminium a la formule suivante :

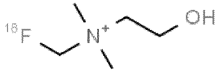

Selon les différents aspects de l'invention, de la choline marquée, préférablement de la [¹⁸F]-Fcholine, est administrée à un patient préalablement à la quantification de la choline marquée chez le patient, de préférence, 10 à 60 minutes préalablement à sa quantification.

L'administration de choline marquée est généralement réalisée par voie intraveineuse mais peut être également réalisée par tout autre mode d'administration approprié.

La dose de choline marquée est déterminée et ajustée en fonction de facteurs tels que la nature, l'âge, le sexe et le poids du patient (e.g. dose de 3,7 MBq/kg, 300 MBq maximum).

Le patient peut être un sujet atteint ou soupçonné d'être atteint d'une fibrose pulmonaire progressive, telle que la fibrose pulmonaire idiopathique.

Le patient peut être un animal humain ou non humain. De préférence, le patient est un mammifère, plus préférablement le patient est un être humain.

Selon les protocoles usuels, lorsque la choline marquée est de la [¹⁸F]-Fcholine et que le patient est un être humain, une dose de 2 à 4 MBq/kg de masse corporelle est administrée.

Avantageusement, la méthode de détection selon l'invention comprend une étape de quantification du signal émis par la choline marquée chez le patient.

Le signal émis par la choline marquée est positivement corrélé à l'activité métabolique de la choline kinase dans le tissu pulmonaire. Sous l'activité de la choline kinase la choline marquée est incorporée à la membrane des cellules. Un fort signal de la choline marquée traduit une forte incorporation de cette dernière dans les cellules et donc une forte activité de la choline kinase. Une augmentation de l'activité métabolique de la choline kinase se retrouve dans la fibrose pulmonaire. Sans être lié à la théorie, les inventeurs font l'hypothèse que cette augmentation de l'activité métabolique peut être due à une synthèse accrue de phosphatidylcholine.

Dans tous les aspects de la présente invention, la quantification se fait préférablement dans une région déterminée du patient. Typiquement, dans le cas où une fibrose pulmonaire est recherchée, la région déterminée est tout ou une partie des poumons du patient.

La méthode de détection peut comprendre une étape de comparaison de la quantité du signal émis par la choline marquée à au moins une valeur de référence, de préférence dite valeur de référence de détection.

Dans un mode de réalisation, la au moins une valeur de référence est la quantité du signal émis par de la choline marquée détectée par la méthode selon l'invention chez un sujet témoin.

Plusieurs valeurs de référence peuvent être utilisées conjointement.

Dans un mode de réalisation, la valeur de référence est une valeur de référence dite basale. La valeur de référence basale est la quantité du signal émis par de la choline marquée détectée chez un sujet sain (i.e. ne présentant pas de fibrose pulmonaire). Une quantité du signal émis par la choline marquée chez le patient supérieure à ladite valeur de référence basale est indicatrice de la présence d'une fibrose pulmonaire.

Dans un mode de réalisation, la au moins valeur de référence est une valeur obtenue chez le patient lors d'une précédente détection de fibrose pulmonaire utilisant de la choline marquée.

Dans un autre mode de réalisation, la au moins une valeur de référence est le signal émis par la choline marquée circulante ou musculaire ou hépatique, de préférence par la choline marquée circulante. Le ratio du signal émis par la choline marquée par rapport à celui de la choline circulante ou musculaire ou hépatique peut alors être déterminé afin de détecter la fibrose pulmonaire.

Ainsi, l'accumulation de choline marquée au niveau pulmonaire peut également être exprimée en ratio du signal choline marquée pulmonaire sur le signal de choline circulant.Les inventeurs ont montré que la quantité de signal émis par la choline marquée est positivement corrélée à la sévérité de la fibrose pulmonaire.

Dans un mode de réalisation, la au moins une valeur de référence est une valeur de référence dite de grade. Chaque valeur de référence de grade donné correspond à un grade de sévérité donné de la fibrose pulmonaire. Chaque valeur de référence de grade donné est la quantité du signal émis par de la choline marquée chez un patient présentant une fibrose pulmonaire de ce grade donné.

Ainsi la méthode de détection selon l'invention permet de détecter une lésion de fibrose pulmonaire progressive mais également d'en déterminer sa sévérité.

La méthode de détection selon l'invention peut être réalisée chez un patient à différents moments dans le temps. Elle permet ainsi de suivre chez ce patient l'évolution de la fibrose pulmonaire et notamment de la sévérité de cette dernière. Ainsi, une augmentation de la quantité de signal émis par la choline marquée est indicatrice d'une aggravation de la sévérité de la fibrose pulmonaire. A contrario, une diminution de la quantité de signal émis par la choline marquée est indicatrice d'une diminution de la sévérité de la fibrose pulmonaire.

Le suivi de l'évolution de la fibrose pulmonaire peut se faire chez un patient auquel un traitement thérapeutique a été administré. Le traitement thérapeutique peut être par exemple de la pirfénidone ou du nintédanib ou tout autre traitement thérapeutique dont on souhaite évaluer l'efficacité sur le développement de la fibrose pulmonaire. Une diminution de la quantité de signal émis par la choline marquée au cours du traitement thérapeutique est indicatrice d'une réponse du patient audit traitement thérapeutique.

De manière avantageuse, la méthode de détection peut également comprendre une étape de génération d'une image à partir de la quantification du signal émis par la choline marquée.

Ainsi, la génération d'une image à partir de la quantification du signal émis par la choline permet de localiser la ou les zones de la région déterminée présentant une fibrose pulmonaire.

L'étape de génération d'une image à partir de la quantification du signal émis par la choline marquée peut se faire à partir de la quantification du signal émis par la choline marquée en lui-même ou à partir de la comparaison de la quantification du signal émis par la choline marquée avec une valeur de référence.

Le matériel d'imagerie utilisé est fonction du type de marqueur que comprend la choline. Selon le mode de réalisation préféré de l'invention où la choline marquée est une [¹⁸F]-Fcholine, l'imagerie est réalisée, par tomographie par émission de positrons (TEP).

Dans tous les aspects de la présente invention, l'imagerie de la choline marquée peut être couplée à une autre imagerie, comme par exemple une imagerie anatomique telle qu'un scanner (CT) ou une imagerie par résonnance magnétique (IRM).

### Méthode de diagnostic d'une fibrose pulmonaire et en particulier de son caractère progressif

Un deuxième aspect de l'invention concerne la choline marquée pour une utilisation dans une méthode de diagnostic *in vivo* d'une fibrose pulmonaire progressive.

L'invention concerne également une méthode de diagnostic d'une fibrose pulmonaire progressive chez un patient comprenant l'administration de choline marquée au dit patient.

L'invention concerne aussi une méthode pour obtenir des données pour aider au diagnostic d'une fibrose pulmonaire progressive comprenant une étape d'imagerie de choline marquée.

En outre, l'invention concerne l'utilisation de choline marquée dans la préparation d'un réactif de diagnostic d'une fibrose pulmonaire progressive.

Les fibroses pulmonaires progressives (FPP) se rencontrent dans un grand nombre de pneumopathies interstitielles diffuses fibrosantes. En effet, les étiologies les plus fréquentes de FPP sont : la fibrose pulmonaire idiopathique, une fibrose pulmonaire non spécifique idiopathique, une fibrose pulmonaire associées à une pneumopathie d'hypersensibilité, une fibrose pulmonaire d'origine iatrogénique, une fibrose pulmonaire secondaire à des connectivités notamment telles que la sclérodermie, une fibrose pulmonaire associée à unearthrite rhumatoïde ou encore une fibrose pulmonaire dans le cadre d'une sarcoïdose.

La PPF peut être une fibrose pulmonaire progressive associée à une polyarthrite rhumatoïde, une fibrose pulmonaire progressive associée à une sclérose systémique, une fibrose pulmonaire progressive associée à une dermatomyosite ou un syndrome des anti-synthétases.

Dans tous les aspects de la présente invention, la fibrose pulmonaire progressive est choisie dans le groupe comprenant la fibrose pulmonaire idiopathique (FPI), la fibrose pulmonaire secondaire à une pneumopathie d'hypersensibilité, une fibrose pulmonaire d'origine iatrogénique, une fibrose pulmonaire secondaire à des connectivités telles que la sclérose systémique, une fibrose pulmonaire associée à de la polyarthrite rhumatoïde ou une fibrose pulmonaire secondaire à une sarcoïdose.

Selon le mode de réalisation préféré de l'invention, la fibrose pulmonaire progressive est une fibrose pulmonaire idiopathique.

Typiquement, la méthode de diagnostic *in vivo* d'une fibrose pulmonaire progressive comprend la détection de fibrose pulmonaire selon la méthode décrite ci-dessus.

Avantageusement, la méthode de diagnostic d'une fibrose pulmonaire progressive comprend :
- une étape de quantification du signal émis par la choline marquée chez un patient dans une région déterminée, la région déterminée étant tout ou une partie des poumons du patient et
- une étape de comparaison de la quantité du signal émis par la choline marquée à une valeur de référence, dite valeur de référence diagnostique.

Préalablement à la quantification de la choline marquée chez le patient, de la choline marquée, préférablement de la [¹⁸F]-Fcholine, est administrée à un patient.

Le patient est un sujet soupçonné d'être atteint d'une fibrose pulmonaire progressive, telle que la fibrose pulmonaire idiopathique.

Selon un mode de réalisation, une quantité de signal émis par la choline marquée supérieure à la valeur de référence diagnostique est indicatrice que le patient est atteint d'une fibrose pulmonaire progressive.

La valeur de référence diagnostique peut être la quantité du signal émis par de la choline marquée détectée chez un sujet sain (i.e. ne présentant pas de fibrose pulmonaire). Une quantité du signal émis par la choline marquée chez le patient supérieure à ladite valeur de référence diagnostique est indicatrice de la présence d'une fibrose pulmonaire et de ce fait que le patient est atteint d'une fibrose pulmonaire progressive.

Le caractère progressif et en particulier la preuve par imagerie de la progression des lésions pulmonaires font partie des critères définissant la fibrose pulmonaire progressive.

Aussi, selon un autre mode de réalisation, la valeur de référence diagnostique correspond à la quantité du signal émis par la choline marquée chez le sujet soupçonné d'être atteint de cette pathologie à un temps donné antérieur, par exemple environ un an avant la date de réalisation de la méthode de diagnostic selon l'invention. Une quantité de signal émis par la choline marquée obtenue par la méthode de diagnostic selon l'invention supérieure à la valeur de référence diagnostique est alors indicatrice que le sujet est atteint d'une fibrose pulmonaire progressive.

Un aspect de l'invention concerne donc de la choline marquée pour une utilisation dans une méthode de diagnostic *in vivo* du caractère progressif d'une fibrose pulmonaire.

L'invention concerne également une méthode de diagnostic du caractère progressif d'une fibrose pulmonaire chez un patient comprenant l'administration de choline marquée au dit patient.

L'invention concerne aussi une méthode pour obtenir des données pour aider au diagnostic du caractère progressif d'une fibrose pulmonaire comprenant une étape d'imagerie de choline marquée.

La méthode de diagnostic peut également comprendre une étape de génération d'une image à partir de la quantification du signal émis par la choline marquée.

Ainsi, la génération d'une image à partir de la quantification du signal émis par la choline permet de localiser la ou les zones de la région déterminée présentant une fibrose pulmonaire. En fonction de l'étendue de la ou les zones présentant une fibrose pulmonaire, le diagnostic de fibrose pulmonaire progressive peut être précisé.

Selon le mode de réalisation préféré de l'invention où la choline marquée est une [¹⁸F]-Fcholine, la technique d'imagerie utilisée est la tomographie par émission de positrons (TEP).

### Méthode de détermination du degré de sévérité d'une fibrose pulmonaire progressive

Un 3ème aspect de l'invention concerne de la choline marquée pour une utilisation dans une méthode de détermination *in vivo* du degré de sévérité d'une fibrose pulmonaire progressive.

L'invention concerne également une méthode de détermination du degré de sévérité d'une fibrose pulmonaire progressive chez un patient comprenant l'administration de choline marquée audit patient.

L'invention concerne aussi une méthode pour obtenir des données pour aider à la détermination du degré de sévérité d'une fibrose pulmonaire progressive comprenant une étape d'imagerie de choline marquée.

En outre, l'invention concerne l'utilisation de choline marquée dans la préparation d'un réactif pour la détermination du degré de sévérité d'une fibrose pulmonaire progressive.

Selon le mode de réalisation préféré de l'invention, la fibrose pulmonaire progressive est une fibrose pulmonaire idiopathique.

Typiquement, la méthode de détermination *in vivo* du degré de sévérité d'une fibrose pulmonaire progressive comprend la détection de fibrose pulmonaire selon la méthode décrite ci-dessus.

Avantageusement, la méthode de détermination du degré de sévérité d'une fibrose pulmonaire progressive comprend :
- une étape de quantification du signal émis par la choline marquée chez un patient dans une région déterminée, la région déterminée étant tout ou une partie des poumons du patient, et
- une étape de comparaison de la quantité du signal émis par la choline marquée à au moins une valeur de référence, dite valeur de référence de grade, chaque valeur de référence de grade correspondant à un grade de sévérité donné de la fibrose pulmonaire progressive.

Une quantité de signal émis par la choline marquée supérieure ou égale à une valeur de référence de grade étant indicatrice que la sévérité de la fibrose pulmonaire progressive est au moins du grade de sévérité donné.

Une quantité de signal émis par la choline marquée inférieure à la valeur de référence de grade étant indicatrice que le degré de sévérité de la fibrose pulmonaire progressive est moindre que le grade de sévérité donné.

Préalablement à la quantification de la choline marquée chez le patient, de la choline marquée, préférablement de la [¹⁸F]-Fcholine, est administrée à un patient.

Le patient est un sujet atteint ou soupçonné d'être atteint d'une fibrose pulmonaire progressive, telle que la fibrose pulmonaire idiopathique. Préférentiellement, le patient est un sujet atteint d'une fibrose pulmonaire progressive, telle que la fibrose pulmonaire idiopathique.

Chaque valeur de référence de grade donné correspond à un grade de sévérité donné de la fibrose pulmonaire. Chaque valeur de référence de grade donné est la quantité du signal émis par de la choline marquée chez un patient témoin présentant une fibrose pulmonaire progressive de ce grade donné.

La méthode de détermination du degré de sévérité peut également comprendre une étape de génération d'une image à partir de la quantification du signal émis par la choline marquée.

Selon le mode de réalisation préféré de l'invention où la choline marquée est une [¹⁸F]-Fcholine, la technique d'imagerie utilisée est la tomographie par émission de positrons (TEP).

### Méthode de suivi de l'évolution d'une fibrose pulmonaire progressive

Un 4^{ème} aspect de l'invention concerne de la choline marquée pour une utilisation dans une méthode de suivi *in vivo* de l'évolution d'une fibrose pulmonaire progressive.

L'invention concerne également une méthode de suivi de l'évolution d'une fibrose pulmonaire progressive chez un patient comprenant l'administration de choline marquée audit patient.

L'invention concerne aussi une méthode pour obtenir des données pour aider au suivi de l'évolution d'une fibrose pulmonaire progressive comprenant une étape d'imagerie de choline marquée.

En outre, l'invention concerne l'utilisation de choline marquée dans la préparation d'un réactif de suivi de l'évolution d'une fibrose pulmonaire progressive.

Typiquement, la méthode de suivi de l'évolution *in vivo* d'une fibrose pulmonaire progressive comprend :
(a) la détection de fibrose pulmonaire selon la méthode décrite ci-dessus chez un patient à un temps donné T-1,
(b) la détection de fibrose pulmonaire selon la méthode décrite ci-dessus chez le patient à un temps donné, T0 étant supérieur à T-1, et
(c) la comparaison de la quantité du signal émis par de la choline marquée à T0 à la quantité du signal émis par de la choline marquée à T-1.

Avantageusement, la méthode de suivi de l'évolution d'une fibrose pulmonaire progressive comprend :
(a) une étape de quantification du signal émis par la choline marquée chez un patient dans une région déterminée, la région déterminée étant tout ou une partie des poumons du patient, à un temps donné T-1,
(b) une étape de quantification du signal émis par la choline marquée chez le patient dans la région déterminée à un temps donné T0, T0 étant supérieur à T-1, et
(c) la comparaison de la quantité du signal émis par de la choline marquée à T0 à la quantité du signal émis par de la choline marquée à T-1.

Une quantité du signal émis par la choline marquée à T0 supérieure à la quantité du signal émis par la choline marquée à T-1 est alors indicatrice d'une aggravation de la fibrose pulmonaire progressive chez ce patient.

Une quantité du signal émis par la choline marquée à T0 inférieure ou égale à la quantité du signal émis par la choline marquée à T-1 est alors indicatrice d'une amélioration de la fibrose pulmonaire progressive chez ce patient.

Une quantité du signal émis par la choline marquée à T0 égale à la quantité du signal émis par la choline marquée à T-1 est alors indicatrice d'une stagnation de la fibrose pulmonaire progressive chez ce patient.

Préalablement à la quantification de la choline marquée chez le patient, de la choline marquée, préférablement de la [¹⁸F]-Fcholine, est administrée à un patient.

Le patient est un sujet atteint ou soupçonné d'être atteint d'une fibrose pulmonaire progressive, telle que la fibrose pulmonaire idiopathique. Préférentiellement, le patient est un sujet atteint d'une fibrose pulmonaire progressive, telle que la fibrose pulmonaire idiopathique.

Le suivi de l'évolution de la fibrose pulmonaire peut se faire chez un patient auquel un traitement thérapeutique a été administré afin de déterminer la réponse du patient à ce dernier.

Aussi, la méthode de suivi peut comprendre une étape d'administration d'un traitement thérapeutique au patient. Le traitement thérapeutique peut être par exemple de la pirfénidone ou du nintédanib ou tout autre traitement thérapeutique dont on souhaite évaluer l'efficacité sur le développement de la fibrose pulmonaire.

Une diminution de la quantité de signal émis par la choline marquée au cours du traitement thérapeutique est indicatrice d'une réponse du patient audit traitement thérapeutique.

La méthode de suivi de l'évolution peut également comprendre une étape de génération d'une image à partir de la quantification du signal émis par la choline marquée.

Ainsi, la génération d'une image à partir de la quantification du signal émis par la choline marquée permet de localiser la ou les zones de la région déterminée présentant une fibrose pulmonaire. En fonction de l'évolution de l'étendue de la ou les zones présentant une fibrose pulmonaire, l'évolution de la fibrose pulmonaire progressive peut être précisée.

Selon le mode de réalisation préféré de l'invention où la choline marquée est une [¹⁸F]-Fcholine, la technique d'imagerie utilisée est la tomographie par émission de positrons (TEP).

### Méthode d'évaluation de l'efficacité d'un traitement thérapeutique contre une fibrose pulmonaire progressive

Un 5^{ème} aspect de l'invention concerne de la choline marquée pour une utilisation dans une méthode d'évaluation *in vivo* de l'efficacité d'un traitement thérapeutique contre une fibrose pulmonaire progressive.

L'invention concerne également une méthode d'évaluation de l'efficacité d'un traitement thérapeutique contre une fibrose pulmonaire progressive chez un patient comprenant l'administration de choline marquée audit patient.

L'invention concerne aussi une méthode pour obtenir des données pour aider à l'évaluation de l'efficacité d'un traitement thérapeutique contre une fibrose pulmonaire progressive comprenant une étape d'imagerie de choline marquée.

En outre, l'invention concerne l'utilisation de choline marquée dans la préparation d'un réactif d'évaluation de l'efficacité d'un traitement thérapeutique contre une fibrose pulmonaire progressive.

Typiquement, la méthode de suivi de l'évolution *in vivo* d'une fibrose pulmonaire progressive comprend :
(a) la détection de fibrose pulmonaire selon la méthode décrite ci-dessus chez un patient avant traitement thérapeutique,
(b) la détection de fibrose pulmonaire selon la méthode décrite ci-dessus chez le patient après un temps donné de traitement thérapeutique, et
(c) la comparaison de la quantité du signal émis par de la choline marquée après un temps donné de traitement thérapeutique à la quantité du signal émis par de la choline marquée avant traitement thérapeutique.

Avantageusement, la méthode d'évaluation *in vivo* de l'efficacité d'un traitement thérapeutique contre une fibrose pulmonaire progressive caractérisée en ce que la méthode comprend :
(a) une étape de quantification du signal émis par la choline marquée chez un patient dans une région déterminée, la région déterminée étant tout ou une partie des poumons du patient, avant traitement thérapeutique,
(b) une étape de quantification du signal émis par la choline marquée chez le patient dans la région déterminée, après un temps donné de traitement thérapeutique et
(c) la comparaison de la quantité du signal émis par de la choline marquée après un temps donné de traitement thérapeutique à la quantité du signal émis par de la choline marquée avant traitement thérapeutique.

Une diminution ou une stagnation de la quantité du signal émis par la choline marquée après un temps donné de traitement thérapeutique est indicatrice de l'efficacité du traitement thérapeutique.

Préalablement à la quantification de la choline marquée chez le patient, de la choline marquée, préférablement de la [¹⁸F]-Fcholine, est administrée à un patient.

Le patient est un sujet atteint d'une fibrose pulmonaire progressive, telle que la fibrose pulmonaire idiopathique.

Avantageusement, la méthode d'évaluation de l'efficacité d'un traitement thérapeutique comprend une étape d'administration d'un traitement thérapeutique au patient.

La quantité du signal émis par de la choline marquée après un temps donné de traitement thérapeutique peut également être comparée à la quantité du signal émis par de la choline marquée après un temps donné de traitement standard de la fibrose pulmonaire progressive tel que la pirfénidone ou le nintédanib chez un patient témoin ayant un profil pathologique similaire au patient ayant reçu le traitement thérapeutique dont on veut évaluer l'efficacité.

La méthode d'évaluation de l'efficacité d'un traitement thérapeutique peut également comprendre une étape de génération d'une image à partir de la quantification du signal émis par la choline marquée.

Selon le mode de réalisation préféré de l'invention où la choline marquée est une [¹⁸F]-Fcholine, la technique d'imagerie préférentiellement utilisée est la tomographie par émission de positrons (TEP).

### FIGURES

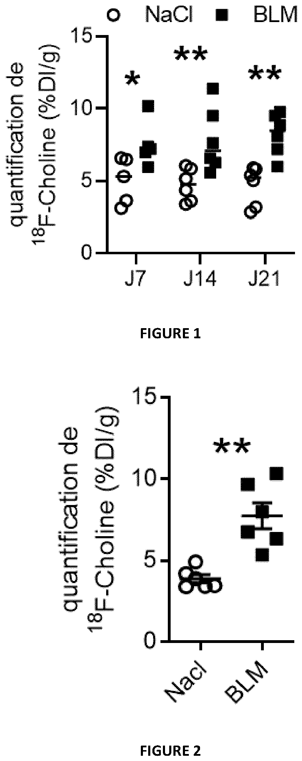

**Figure 1****.** La figure 1 montre la quantification via imagerie PET au [¹⁸F]-Fcholine de zones pulmonaires de souris traitées avec du NaCl (rond blanc) ou de la bléomycine (BLM, carré noir). La quantité de [¹⁸F]-Fcholine est exprimée en pourcentage de la dose injectées (DI) par poids (g). L'analyse statistique a été réalisée par le test de Mann Whitney.
**Figure 2****.** La figure 2 montre la quantification de l'accumulation pulmonaire de [¹⁸F]-Fcholine par comptage gamma de souris traitées avec du NaCl (rond blanc) ou de la bléomycine (BLM, carré noir). L'analyse statistique a été réalisée par le test de Mann Whitney.

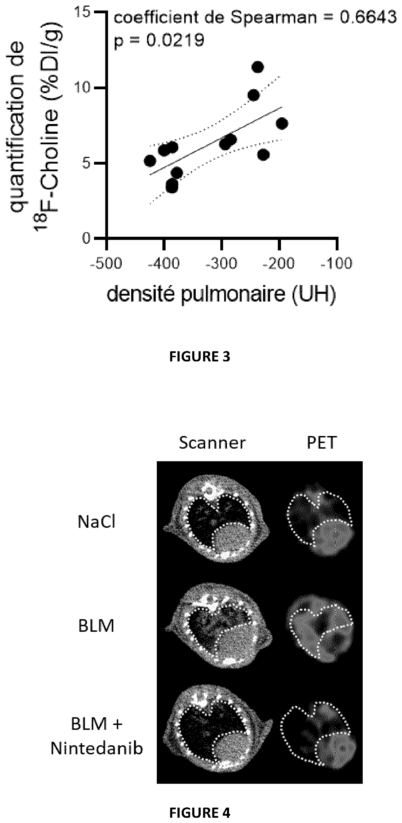

**Figure 3****.** La figure 3 montre la corrélation du signal [¹⁸F]-Fcholine à J14 en fonction de l'atteinte fibrotique mesurée par scanner. La densité pulmonaire est exprimée en unité de Hounsfield (UH). L'analyse statistique a été réalisée par corrélation non paramétrique avec des calculs du coefficient de corrélation de Spearman *: p<0.05,** : p<0.01.
**Figure 4****.** La figure 4 montre des images transversales représentatives (n=6 par groupe) provenant d'imagerie par PET-scanner après injection de [¹⁸F]-Fcholine à des souris traitées avec de la bléomycine, avec ou sans ninténanib, ou du NaCl.

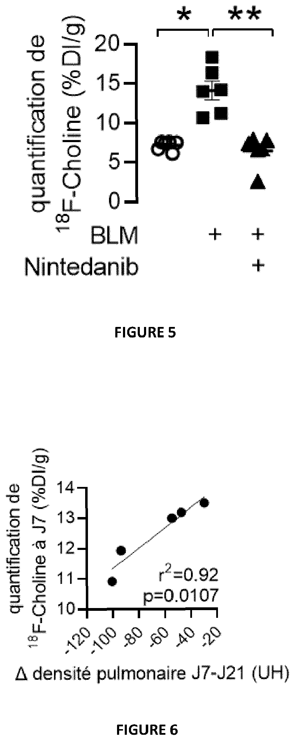

**Figure 5****.** La figure 5 montre la quantification via imagerie PET au [¹⁸F]-Fcholine de zones pulmonaires de souris traitées avec du NaCl à J0 (rond blanc), de la bléomycine à J0 (BLM, carré noir) ou une combinaison de bléomycine à J0 et de nintédanib de J8 à J20 (triangle noir). L'analyse statistique a été réalisée par le test de Mann Whitney.
**Figure 6****.** La figure 6 montre la corrélation du signal [¹⁸F]-Fcholine à J7 en fonction de l'atteinte pulmonaire entre J7 et J21. La densité pulmonaire est exprimée en unité de Hounsfield (UH). L'analyse statistique a été réalisée par corrélation non paramétrique avec des calculs du coefficient de corrélation de Spearman * :p<0.05,** : p<0.01.

### EXEMPLES

### Exemple 1 - Modèle de fibrose pulmonaire induite par bléomcyine

### Matériel et méthodes

Une fibrose pulmonaire a été induite chez des souris par administration de bléomycine. Ainsi, des souris C57Bl/6 mâles de 8 semaines ont été exposées à de la bléomycine (BLM, n=6) à la dose de 1.25mg/kg par aspiration oropharyngée. A titre de contrôle, des souris C57Bl/6 mâles de 8 semaines ont été traitées par injection par voie intraveineuse de sérum physiologique (NaCl, n=6).

Les souris ont ensuite été suivies à 7, 14 et 21 jours après leur traitement à la bléomycine ou au NaCl. A chacun de ces temps, il a été injecté à l'ensemble des souris du [¹⁸F]-Fcholine (5 MBq dans 100µl par souris, par injection intracaudale) avant de subir une double imagerie tomographie par émission de positons (TEP)- scanner thoracique (CT).

L'injection de la [¹⁸F]-Fcholine a lieu 10 à 20 minutes avant l'imagerie TEP. L'imagerie TEP centrée sur les poumons dure 30 minutes (250-700 keV). Pendant la période de distribution de la [¹⁸F]-Fcholine un scanner thoracique (CT) est réalisé (150µA, 45kV, 360 projections, 2 shots/projection). Durant la procédure, les animaux sont maintenus sous anesthésie (Isofurane 1.5%) sur un lit chauffant (37°C).

Toutes les images de fusion TEP/CT sont obtenues à l'aide du logiciel VivoQuant^{™} (Invicro, USA).

Chaque image TEP/CT est interprétée visuellement et les régions d'intérêt 3D (3DROI) correspondant aux poumons sont dessinées semi-automatiquement pour la quantification de la tomographie et pour déterminer leur contenu en radioactivité. Les doses injectées par animal sont mesurées au moment de l'injection en MBq. Le contenu en radioactivité des poumons est exprimé en MBq, converti en pourcentage de la dose injectée par gramme de tissu (%ID/g). Le scanner TEP est étalonné de manière croisée avec le calibrateur de dose et les données TEP sont corrigées pour la diffusion et l'atténuation. Toutes les images sont corrigées pour la quantification.

### Résultats

La quantification du signal [¹⁸F]-Fcholine a montré un signal significativement plus élevé dans les poumons des souris atteintes de fibrose pulmonaire en comparaison aux souris contrôles (Fig. 1).

La donnée de quantification sur imagerie a été confirmée par comptage gamma à J21 (Figure 2).

La différence de signal [¹⁸F]-Fcholine entre les souris atteintes de fibrose pulmonaire et les souris contrôles est observée pour tous les temps post-blémoycine et le signal s'accumule dans des zones pulmonaires non aérées (tissu fibreux). De plus, le signal [¹⁸F]-Fcholine à J14 est corrélé à la sévérité de la fibrose mesurée par scanner thoracique à J21 (Figure 3). Cette expérience a été répétée avec un groupe supplémentaire de souris exposées à la bléomycine et recevant du nintédanib (n=6 souris pour chaque groupe). Cette seconde expérimentation a confirmé les données acquises lors de la première expérience concernant la différence de signal [¹⁸F]-Fcholine entre les souris traitées au sérum physiologique (NaCl) et à la bléomycine (BLM) (Figures 4 et 5). De plus, chez les animaux avec fibrose pulmonaire et recevant du nintédanib, le signal [¹⁸F]-Fcholine est corrélé avec la sévérité de l'atteinte fibrotique évaluée par scanner thoracique (Figure 6).

## Revendications

1. Méthode non invasive de détection *in vivo* de lésion(s) de fibrose pulmonaire utilisant de la choline marquée **caractérisée en ce qu'**elle comprend :
- une étape de quantification du signal émis par la choline marquée chez un patient dans une région déterminée, la région déterminée étant tout ou une partie des poumons du patient.

2. Méthode non invasive de détection selon la revendication 1 **caractérisée en ce qu'**elle comprend une étape de génération d'une image à partir de la quantification du signal émis par la choline marquée.

3. Utilisation de choline marquée en tant qu'agent d'imagerie afin de détecter *in vivo* des lésions de fibrose pulmonaire **caractérisée en ce qu'**elle comprend :
- une étape de quantification du signal émis par la choline marquée chez un patient dans une région déterminée, la région déterminée étant tout ou une partie des poumons du patient,
- une étape de génération d'une image à partir de la quantification du signal émis par la choline marquée.

4. Choline marquée pour une utilisation dans une méthode de diagnostic *in vivo* d'une fibrose pulmonaire progressive **caractérisée en ce que** la méthode comprend :
- une étape de quantification du signal émis par la choline marquée chez un patient dans une région déterminée, la région déterminée étant tout ou une partie des poumons du patient et
- une étape de comparaison de la quantité du signal émis par la choline marquée à une valeur de référence, dite valeur de référence diagnostique,
- une quantité de signal émis par la choline marquée supérieure à la valeur de référence diagnostique étant indicatrice que le patient est atteint d'une fibrose pulmonaire progressive.

5. Choline marquée pour une utilisation dans une méthode de détermination *in vivo* du degré de sévérité d'une fibrose pulmonaire progressive **caractérisée en ce que** la méthode comprend :
- une étape de quantification du signal émis par la choline marquée chez un patient dans une région déterminée, la région déterminée étant tout ou une partie des poumons du patient, et
- une étape de comparaison de la quantité du signal émis par la choline marquée à au moins une valeur de référence, dite valeur de référence de grade, chaque valeur de référence de grade correspondant à un grade de sévérité donné de la fibrose pulmonaire progressive
- une quantité de signal émis par la choline marquée supérieure ou égale à une valeur de référence de grade étant indicatrice que la sévérité de la fibrose pulmonaire progressive est au moins du grade de sévérité donné,
- une quantité de signal émis par la choline marquée inférieure à la valeur de référence de grade étant indicatrice que le degré de sévérité de la fibrose pulmonaire progressive est moindre que le grade de sévérité donné.

6. Choline marquée pour une utilisation dans une méthode de suivi *in vivo* de l'évolution d'une fibrose pulmonaire progressive **caractérisée en ce que** la méthode comprend :
(a) une étape de quantification du signal émis par la choline marquée chez un patient dans une région déterminée, la région déterminée étant tout ou une partie des poumons du patient, à un temps donné T-1,
(b) une étape de quantification du signal émis par la choline marquée chez le patient dans la région déterminée à un temps donné T0, T0 étant supérieur à T-1, et
(c) la comparaison de la quantité du signal émis par de la choline marquée à T0 à la quantité du signal émis par de la choline marquée à T-1,
- une quantité du signal émis par la choline marquée à T0 supérieure à la quantité du signal émis par la choline marquée à T-1 est alors indicatrice d'une aggravation de la fibrose pulmonaire progressive chez ce patient,
- une quantité du signal émis par la choline marquée à T0 inférieure ou égale à la quantité du signal émis par la choline marquée à T-1 est alors indicatrice d'une amélioration de la fibrose pulmonaire progressive chez ce patient,
- une quantité du signal émis par la choline marquée à T0 égale à la quantité du signal émis par la choline marquée à T-1 est alors indicatrice d'une stagnation de la fibrose pulmonaire progressive chez ce patient.

7. Choline marquée pour une utilisation dans une méthode d'évaluation *in vivo* de l'efficacité d'un traitement thérapeutique d'une fibrose pulmonaire progressive **caractérisée en ce que** la méthode comprend :
(a) une étape de quantification du signal émis par la choline marquée chez un patient dans une région déterminée, la région déterminée étant tout ou une partie des poumons du patient, avant traitement thérapeutique,
(b) une étape de quantification du signal émis par la choline marquée chez le patient dans la région déterminée, après un temps donné de traitement thérapeutique et
(c) la comparaison de la quantité du signal émis par la choline marquée après un temps donné de traitement thérapeutique à la quantité du signal émis par la choline marquée avant traitement thérapeutique,
- une diminution ou une stagnation de la quantité du signal émis par la choline marquée après un temps donné de traitement thérapeutique est indicatrice de l'efficacité du traitement thérapeutique.

8. Méthode de détection selon la revendication 1 ou 2, utilisation de choline marquée selon la revendication 3 et/ou choline marquée pour une utilisation selon l'une quelconque des revendications 4 à 7 **caractérisée**(s) en ce que la choline marquée comprend un marqueur choisi dans le groupe consistant en un marqueur fluorescent, un marqueur radioactif et un marqueur paramagnétique.

9. Méthode de détection selon la revendication 1 ou 2, utilisation de choline marquée selon la revendication 3 et/ou choline marquée pour une utilisation selon l'une quelconque des revendications 4 à 7 **caractérisée**(s) en ce que la choline marquée est de la [¹⁸F]-Fcholine.

10. Choline marquée pour une utilisation selon l'une quelconque des revendications 4 à 7, 8 ou 9 **caractérisée en ce que** la fibrose pulmonaire progressive est choisie dans le groupe consistant en une fibrose pulmonaire idiopathique, une fibrose pulmonaire non spécifique idiopathique, une fibrose pulmonaire associée à une pneumopathie d'hypersensibilité, une fibrose pulmonaire d'origine iatrogénique, une fibrose pulmonaire secondaire à des connectivités, une fibrose pulmonaire associée à une polyarthrite rhumatoïde une fibrose pulmonaire associée à une sarcoïdose, une fibrose pulmonaire associée à une sclérose systémique et une fibrose pulmonaire associée à une dermatomyosite ou un syndrome des anti-synthétases.
